# EUROPEAN PATENT APPLICATION

(11) **EP 1 293 209 A1**
(43) Date of publication of application: **19.03.2003**
(21) Application number: 02256156.7
(22) Date of filing: 05.09.2002
(51) Int. Cl.: A61K 31/485, A61K 31/515, A61K 47/48

(54) **Oral pharmaceutical dosage forms with reduced potential for drug abuse**

(30) Priority: 17.09.2001 US 322768 P; 02.11.2001 US 16415
(71) Applicant: ROHM AND HAAS COMPANY, Philadelphia, Pennsylvania 19106-2399 (US)
(72) Inventor: Hughes, Lyn, Harleysville, Pennsylvania 19438 (US)
(74) Representative: Kent, Venetia Katherine

(57) **Abstract**

Solid oral dosage forms of controlled substances are described that have reduced potential for abuse by inhalation, mastication, and injection .

## Description

### BACKGROUND

Abuse of controlled substances is a serious and growing problem throughout the world. For example, the abuse of an extended release form of oxycodone has been the recent subject of many articles such as 'Playing With Pain Killers' and 'How One Town Got Hooked'. See, Newsweek, April 9, 2001, pages 45-51. Further, The New York Times profiled the problem of oxycodone abuse by inhalation of the crushed pill. See, The New York Times, July 29, 2001. It is estimated that in America four million people over the age of 12 used prescription painkillers and stimulants for non-medical reasons in the space of just one month, approximately half of them saying they'd done it for the first time. Emergency room visits related to such abuse approximately doubled between 1992 and 1999.

There are three main routes that drug abusers use for administering the drug substances: parenteral, oral, and inhalation. The parenteral route is commonly called 'mainlining' and requires the drug substance to be in solution such that it can be injected intravenously with a syringe. For solid dosage form drugs this requires some type of extraction and concentration procedure to render the drug substance suitable for injection. Inhalation of a solid drug substance through the nose is commonly called 'snorting'. For solid dosage form drugs this requires only that the dosage form be crushed into a powder, or emptied from a capsule. Breathing in vapors is frequently known as 'huffing'. Both snorting and huffing result in the rapid absorption of the drug substance through the mucosa of the respiratory system.

The potential for abuse is increased by the use of extended release formulations because they typically contain more than the immediate release single dose of active ingredient. Circumventing the extended release mechanism delivers the full dose, which is intended to be delivered over a longer time period, immediately. For example, crushing an extended release oxycodone tablet separates a gelling matrix from the oxycodone active ingredient, such that when inhaled through the nose the gelling matrix cannot exert the extended release effect. Similarly it is sometimes possible to circumvent the extended release effect by chewing the dosage form.

The use of coatings to extend the release of drug substances is very well known in the art. See, Remington's Pharmaceutical Sciences, 16^{th} Edition, Chapter 90. Such dosage forms are also subject to said modes of abuse because the coating can be damaged by crushing or chewing.

WO0108661 describes an extended release dosage form of opioids that uses an ion exchange resin. This dosage form is also subject to said modes of abuse because the ion exchange resin and the active ingredient can be separated by crushing.

Various methods have been used to reduce the potential for abuse of controlled substances. These methods have focused on the parenteral and oral routes of administration.

US3,773,955, US3,966,940, and US4,457,933 describe oral dosage forms containing a combination of opioid agonists and antagonists, in which the effect of the antagonist when administered according to the correct procedure does not affect the therapeutic pain management value of the agonist. However, when the agonist and antagonist are extracted for parenteral administration by an addict the effect of the agonist desired by the addict is decreased. This approach was further adopted in WO9004965 where it was incorporated into a transdermal delivery device, and in US 6,228,863 where it was developed into a dosage form from which the agonist could not be separated from the antagonist except by using a sophisticated multi-step procedure.

In US 3,980,766 multiple methods for reducing abuse potential are described. One method is to include a thickening agent such that the concentrated extract containing the drug can not be injected with a syringe. Another is the incorporation of agents that cause the precipitation of the drug during isolation, thus rendering it unsuitable for injection. The addition of a thickener has also been used in US 4,070,494, WO9107950, and WO9520947.

In WO0033835 additives are included in the dosage forms such that when added to drinks create a visible change in the drink. This invention reduces the potential for abuse by oral administration of the substance by one person to another without their knowledge.

However, none of the cited references solve the problem of potential abuse via inhalation or mastication of an oral dosage form. Applicants have surprisingly discovered an oral dosage form useful in reducing the potential for drug abuse via inhalation, mastication, or injection of illicit extracts of said oral dosage form.

The term "therapeutic concentration" as used herein means the concentration of the pharmaceutically active ingredient in the blood plasma that is obtained by the administration of the recommended doses using the prescribed method of administration. Recommended doses for Schedule II - V controlled substances are defined in the literature. For example, see 'Drug Facts and Comparisons', published by Facts and Comparisons, St Louis.

The term "high," as used herein means the non-therapeutic effect desired by drug addicts and recreational drug users

The term "mucosal membrane" as used herein means any mucosal membrane of the body through which an active substance can be administered, including, but not limited to, nasal, lingual, buccal, pharyngeal, bronchial, rectal, urethral and vaginal.

The term "respiratory mucosal membrane" as used herein means the mucous membrane lining the nasal and pharyngeal cavities, the bronchial tubes, and the lungs. Typically, snorting into the nasal cavity is the common, preferred route of abuse for a solid oral dosage form which has been crushed by one intending to inhale said crushed dosage form to obtain the high.

The term "illicit extracts" as used herein are those extracts obtained by any of the means known to drug addicts, drug users, and recreational drug users for extracting an active substance from an oral dosage form. In the interests of social responsibility these methods will not be described herein.

The term "sensory agent" as used herein means those agents that modify ones sensory perception of the dosage form.

The term "meq/g", as used herein, refers to the fact that ion exchange resins are characterized by their capacity to exchange ions. This is expressed as the "Ion Exchange Capacity." For cation exchange resins the term used is "Cation Exchange Capacity," and for anion exchange resins the term used is "Anion Exchange Capacity." The ion exchange capacity is measured as the number equivalents of an ion that can be exchanged and can be expressed with reference to the mass of the polymer ( herein abbreviated to "Weight Capacity") or its volume (often abbreviated to "Volume Capacity"). A frequently used unit for weight capacity is "milliequivalents of exchange capacity per gram of dry polymer." This is commonly abbreviated to "meq/g."

### SUMMARY OF THE INVENTION

The present invention relates to an oral pharmaceutical dosage form not susceptible to abuse by mucosal membrane administration providing a plasma concentration that does not exceed the therapeutic concentration.

The present invention also relates to an oral pharmaceutical dosage form not susceptible to abuse by respiratory mucosal membrane administration providing a plasma concentration that does not exceed the therapeutic concentration.

The present invention further relates to an oral pharmaceutical dosage form not susceptible to abuse by mastication providing a plasma concentration that does not exceed the therapeutic concentration.

Finally, the present invention relates to an oral pharmaceutical dosage form not susceptible to abuse by injection of an illicit extract of said oral dosage form providing a plasma concentration that does not exceed the therapeutic concentration.

### DETAILED DESCRIPTION OF THE INVENTION

The present invention relates to an oral pharmaceutical dosage form not susceptible to abuse by mucosal membrane administration providing a plasma concentration that does not exceed the therapeutic concentration.

The present invention also relates to an oral pharmaceutical dosage form not susceptible to abuse by respiratory mucosal membrane administration providing a plasma concentration that does not exceed the therapeutic concentration.

The present invention further relates to an oral pharmaceutical dosage form not susceptible to abuse by mastication providing a plasma concentration that does not exceed the therapeutic concentration.

Finally, the present invention relates to an oral pharmaceutical dosage form not susceptible to abuse by injection of an illicit extract of said oral dosage form providing a plasma concentration that does not exceed the therapeutic concentration.

Specifically, the dosage form of the present invention renders the controlled substance unable to deliver the desired non therapeutic effect, i.e., the high.

The utility of the invention lies in the fact that the rate of release of the controlled substance is not affected by crushing the solid oral dosage form, or of emptying the dosage from a capsule. For example, when said solid oral dosage form is crushed and inhaled, chewed or illicitly extracted and injected for non-therapeutic purposes the controlled substance is not available for total release, but will release at a rate that does not result in a plasma concentration that exceeds the therapeutic concentration. The high is not obtained at the therapeutic concentration.

The Controlled Substances Act of 1970 regulates the manufacturing, distribution, and dispensing of drugs that have abuse potential. The Drug Enforcement Agency (DEA) within the US Department of Justice is the chief agency responsible for enforcing said act. Drugs under the jurisdiction of said Act are divided into five schedules (I thru V) based on their medical utility, potential for abuse, and physical and psychological dependence. Schedule I substances have high abuse potential and no accepted medical use. Schedule II also have high abuse potential, but also have medical utility. Schedules III, IV, and V have progressively lower abuse potential.

Because the DEA rates abuse potential based on specific dosage forms it is not uncommon for a drug to be rated in multiple schedules. For example codeine appears as Schedule II, Schedule III, and Schedule IV, depending on the specific dosage form and dosage amount. To avoid duplication in the list of controlled substance below, multiple occurrences have been removed and any controlled substance that had multiple occurrences is placed in the highest abuse potential category for which is has been scheduled. For example, codeine has been included as Schedule II, but not Schedule III or Schedule IV. This is not intended to limit the scope of the invention. The utility of the Applicant's invention lies in the fact that any ionizable controlled substance, regardless of what schedule it appears on, is suitable for formulating into the Applicant's dosage form.

Controlled substances useful in the practice of the invention are those categorized by the DEA as Schedule II, III, IV, and V controlled substances. Controlled substances useful in the practice of the invention must be ionizable such that a controlled substance-ion exchange resin complex can be formed. Schedule II substances include, but are not limited to, 1-Phenylcyclohexylamine, 1-Piperidinocyclohexanecarbonitrile, Alfentanil, Alphaprodine, Amobarbital, Amphetamine, Anileridine, Benzoylecgonine, Bezitramide, Carfentanil, Cocaine, Codeine, Dextropropoxyphen, Dihydrocodeine, Diphenoxylate, Diprenorphine, Ecgonine, Ethylmorphine, Etorphine HCl, Fentanyl, Glutethimide, Hydrocodone, Hydromorphone, Isomethadone, Levo-alphacetylmethadol, Levomethorphan, Levorphanol, Meperidine, Metazocine, Methadone, Methamphetamine, Methylphenidate, Metopon, Morphine, Nabilone, Oxycodone, Oxymorphone, Pentobarbital, Phenazocine, Phencyclidine, Phenmetrazine, Piminodine, Racemethorphan, Racemorphan, Remifentanil, Secobarbital, Sufentanil, Thebaine

Schedule III substances include, but are not limited to, Aprobarbital, Barbituric acid derivative, Benzphetamine, Butabarbital, Butalbital, Chlorphentermine, Ketamine, Lysergic acid, Lysergic acid amide, Nalorphine, Phendimetrazine, Talbutal, Thiamylal, Thiopental, , Vinbarbital.

Schedule IV substances include, but are not limited to, Alprazolam, Barbital, Bromazepam, Butorphanol, Camazepam, Cathine, Chloral, Chlordiazepoxide, Clobazam, Clonazepam, Clorazepate, Clotiazepam, Cloxazolam, Delorazepam, Dexfenfluramine, Diazepam, Diethylpropion, Difenoxin, Estazolam, Ethyl loflazepate, Fencamfamin, Fenfluramine, Fenproporex, Fludiazepam, Flunitrazepam, Flurazepam, Halazepam, Haloxazolam, Ketazolam, Loprazolam, Lorazepam, Lormetazepam, Mazindol, Medazepam, Mefenorex, Methohexital, Methylphenobarbital, Midazolam, Nimetazepam, Nitrazepam, Nordiazepam, Oxazepam, Oxazolam, Pemoline, Pentazocine, Phenobarbital, Phentermine, Pinazepam, Pipradrol, Prazepam, Quazepam, Sibutramine, Temazepam, Tetrazepam, Triazolam, Zaleplon, Zolpidem.

Schedule V substances include, but are not limited to, Buprenorphine, Difenoxin, Pyrovalerone.

Preferred controlled substances useful in the practice of the invention are those categorized by the DEA as Schedule II, III, and IV controlled substances.

More preferred controlled substance useful in the practice of the invention are those categorized by the DEA as Schedule II and III controlled substances.

Most preferred controlled substance useful in the practice of the invention are those categorized by the DEA as Schedule II controlled substances. The most preferred Schedule II substance is oxycodone.

The oral dosage form of the present invention is prepared by making a complex of a controlled substance and an ion exchange resin and formulating said complex into an oral dosage form.

The controlled substance-ion exchange resin complex can by formulated into any of the oral dosage forms known in the art including, but not limited to, powders, tablets, pills, and capsules.

The controlled substance-ion exchange resin complex can be prepared by any of the methods known in the art. The typical method, known to those skilled in the art, for loading ionizable substances onto an ion exchange resin to form the ionizable substance-ion exchange resin complex is to dissolve an acidic or basic, ionizable substance in water, and then mix it with a suitable ion exchange resin. See, for example, US2,990,332.

Ion exchange resins useful in the practice of the present invention include, but are not limited to, anion exchange resins and cation exchange resins. Preferably, said resins are suitable for human ingestion.

Preferred anion exchange resins include, but are not limited to, styrenic strongly basic anion exchange resins with a quaternary amine functionality having a weight capacity of 0.1 to 15 meq/g, and styrenic weakly basic anion exchange resins with a primary, secondary, or tertiary amine functionality having a weight capacity of 0.1 to 8.5 meq/g, and acrylic or methacrylic strongly basic anion exchange resins with a quaternary amine functionality having a weight capacity of 0.1 to 12 meq/g, and acrylic or methacrylic weakly basic anion exchange resins with a primary, secondary, or tertiary amine functionality having a weight capacity of 0.1 to 12 meq/g, and allylic and vinylic weakly basic anion exchange resins with a primary, secondary, or tertiary amine functionality having a weight capacity of 0.1 to 24 meq/g, that are suitable for human and animal ingestion.

Most preferred anion exchange resins include, but are not limited to, styrenic anion exchange resins with quaternary amine functionality with weight capacity of 0.1 to 6 meq/g and acrylic anion exchange resins with tertiary amine functionality with weight capacity of 0.1 to 12 meq/g, that are suitable for human and animal ingestion.

Preferred cation exchange resins include, but are not limited to, styrenic strongly acidic cation exchange resins with sulfonic or phosphonic acid functionalities having a weight capacity of 0.1 to 8 meq/g; and styrenic weakly acidic cation exchange resins with carboxylic or phenolic acid functionalities having a weight capacity of 0.1 to 8.5 meq/g; and acrylic or methacrylic weakly acidic cation exchange resins with a carboxylic or phenolic acid functionality with a weight capacity of 0.1 to 14 meq/g, that are suitable for human and animal ingestion.

Most preferred cation exchange resins include, but are not limited to, styrenic weakly acidic cation exchange resin with a phenolic functionality with a weight capacity of 0.1 to 8.5 meq/g; and a styrenic strongly acidic cation exchange resin with a sulfonic acid functionality with weight capacity of 0.1 to 8 meq/g, or a methacrylic weakly acidic cation exchange resin with a carboxylic acid functionality with weight capacity of 0.1 to 12 meq/g.

Ion exchange resins useful in the practice of this invention have a moisture content between 0% and the water retention capacity of said resin.

Ion exchange resins useful in this invention are in powder or whole bead form.

Strongly acidic and weakly acidic cation exchange resins useful in the practice of this invention are in the acid form or salt form or partial salt form.

Strongly basic anion exchange resins useful in the practice of this invention are in the salt form.

Weakly basic anion exchange resins useful in the practice of this invention are in the free-base form or salt form.

In addition to the controlled substance-ion exchange resin complex, excipients are used in the manufacture of the oral dosage forms of the present invention. Excipients useful in the practice if this invention include, but are not limited to, preservatives, viscosity agents, fillers, lubricants, glidants, disintegrants, binders, coatings, and sensory agents.

Preferred preservatives include, but are not limited to, phenol, alkyl esters of parahydroxybenzoic acid, o-phenylphenol benzoic acid and the salts thereof, boric acid and the salts thereof, sorbic acid and the salts thereof, chlorobutanol, benzyl alcohol, thimerosal, phenylmercuric acetate and nitrate, nitromersol, benzalkonium chloride, cetylpyridinium chloride, methyl paraben, and propyl paraben. Particularly preferred are the salts of benzoic acid, cetylpyridinium chloride, methyl paraben and propyl paraben. The compositions of the present invention generally include from 0-2% preservatives.

Preferred viscosity agents include, but are not limited to, methylcellulose, sodium carboxymethylcellulose, hydroxypropyl-methylcellulose, hydroxypropylcellulose, sodium alginate, carbomer, povidone, acacia, guar gum, xanthan gum and tragacanth. Particularly preferred are methylcellulose, carbomer, xanthan gum, guar gum, povidone, sodium carboxymethylcellulose, and magnesium aluminum silicate. Compositions of the present invention include 0-25% viscosity agents.

Preferred fillers include, but are not limited to, lactose, mannitol, sorbitol, tribasic calcium phosphate, dibasic calcium phosphate, compressible sugar, starch, calcium sulfate, dextro and microcrystalline cellulose. The compositions of the present invention contain from 0-75% fillers.

Preferred lubricants include, but are not limited to, magnesium stearate, stearic acid, and talc. The pharmaceutical compositions of the present invention include 0-2% lubricants.

Preferred glidants include, but are not limited to, talc and colloidal silica. The compositions of the present invention include from 0-5% glidants.

Preferred disintegrants include, but are not limited to, starch, sodium starch glycolate, crospovidone, croscarmelose sodium, polacrilin potassium, and microcrystalline cellulose. The pharmaceutical compositions of the present invention include from 0-30% disintegrants.

Preferred binders include, but are not limited to, acacia, tragacanth, hydroxypropylcellulose, pregelatinized starch, gelatin, povidone, hydroxypropylcellulose, hydroxypropyl-methylcellulose, methylcellulose, sugar solutions, such as sucrose and sorbitol, and ethylcellulose. The compositions of the present invention include 0.1-10% binders.

Further, the controlled substance-ion exchange resin complex can be coated to produce beneficial effects other than extended release of the controlled substance. The use of coatings for such purposes is well known in the art. See Remington's Pharmaceutical Sciences, 16^{th} Edition, Chapter 90.

Sensory agents useful in the practice of the present invention include, but are not limited to, bitter agents such as denatonium benzoate (Bitrex®), sour agents such as citric acid, pleasant tasting agents such as orange oil, pleasant smelling agents such as mint, and spicy or irritating agents such as capsaicin.

The following non limiting examples illustrate the present invention:

### EXAMPLE 1 Preparation of an oxycodone-ion exchange resin complex.

40g of oxycodone hydrochloride, a Schedule II controlled substance is dissolved in 2000ml of water. 110g of a powdered cation exchange resin with sulfonic acid functionality in the sodium form is then added to this solution, and the resulting mixture is shaken for at least 12 hours. The mixture is filtered using a Buchner funnel with a filter capable of retaining particles >0.003mm. The wet-cake is washed in place with 1000ml of water. The wet-cake is dried in a vacuum oven at 60°C for 15 hours, or until constant weight is reached, to give the oxycodone-ion exchange resin complex.

### EXAMPLE 2 Preparation of a codeine-ion exchange resin complex.

Codeine sulfate (30g), a Schedule II controlled substance, is dissolved in 1400ml of water. To the codeine sulfate solution is added 170g of a powdered cation exchange resin with sulfonic acid functionality in the sodium form. The mixture is then mixed for 4 hours, forming a codeine-ion exchange resin complex suspension. The suspension is filtered using a screen centrifuge to remove the water and then dried in a fluid bed dryer by passing air at 50°C at a rate sufficient to fluidize the complex.

### EXAMPLE 3 Preparation of a tablet of the present invention

| **Composition** | |
|---|---|
| Oxycodone-ion exchange resin complex (Example 1) | 300.0g |
| Lactose (ground) | 35.0g |
| Colloidal silica | 3.0g |
| Polyvinylpyrrolidone | 3.0g |
| Microcrystalline cellulose | 40.0g |
| Corn starch | 69.0g |

All the solid ingredients are passed through a 0.6mm sieve and mix together. The mixture is used to make 9mm diameter tablets by compression. Each tablet weighs 230mg and contains an amount of active ingredient equivalent to 40mg of oxycodone hydrochloride.

### EXAMPLE 4 Preparation of a capsule of the present invention

2000g of the codeine-ion exchange resin complex obtained in Example 2 is filled into 10,000 size 1 capsules. Each capsule contains an amount of active ingredient equivalent to 30mg of codeine sulfate.

### EXAMPLE 5 Preparation of pentobarbital-ion exchange resin complex

100g of pentobarbital sodium, a Schedule II controlled substance, is dissolved in 5000ml of water. 100g of a powdered anion exchange resin with quarternary amine functionality in the chloride form is then added to this solution, and the resulting mixture is shaken for at least 12 hours. The mixture is filtered using a Buchner funnel with a filter capable of retaining particles >0.003mm. The wet-cake is washed in place with 2000ml of water. The wet-cake is dried in a vacuum oven at 60°C for 15 hours, or until constant weight is reached, to give the pentobarbital-ion exchange resin complex.

### EXAMPLE 6 Preparation of a tablet of the present invention

| **Composition per tablet** | |
|---|---|
| Pentobarbital-ion exchange resin complex (Example 5) | 200mg |
| Lactose (spray-dried) | 200mg |
| Magnesium stearate | 10mg |
| Starch | 90mg |

The complex is passed through a 0.6mm sieve, and is then mixed with the lactose in a twinblade mixer for approximately 20 minutes. The starch and magnesium stearate are then added and the mixing continued for a further 15 minutes. The blend is then compressed into tablets on a standard tablet press to give 500mg tablets each containing the equivalent of 100mg of pentobarbital sodium.

### EXAMPLE 7

A Caucasian female addict, age 27, weighing 45 kg crushes two 40 mg oxycodone tablets, as prepared in Example 3, into a powder and administers said powder through her nasal cavity. The non-therapeutic effect, i.e. the high , is not obtained.

### EXAMPLE 8

At a party a Caucasian male recreational drug user, aged 17, weighing 65 kg is offered two 40mg oxycodone tablets, as prepared in Example 3, and chews them. The non-therapeutic effect, i.e. the high , is not obtained.

### EXAMPLE 9

An African-American male, aged 18, weighing 66 kg, who has never abused drugs breaks a 30mg codeine capsule, empties out the powder from within said capsule, as prepared in Example 4, and administers said powder through his nasal cavity for the first time. The non-therapeutic affect, i.e. the high, is not obtained.

### EXAMPLE 10

An Asian female recreational drug user, aged 28, weighing 55 kg, crushes a 100mg pentobarbital tablet, as prepared in Example 6, to a powder and administers said powder through her nasal cavity. The non-therapeutic effect, i.e. the high, is not obtained.

### EXAMPLE 11

A Caucasian male addict, age 48, prepares an illicit extract of two 40mg oxycodone tablets as prepared in Example 3. He then injects the extract intravenously. The non-therapeutic effect, i.e., the high, is not obtained.

## Claims

1. An oral pharmaceutical dosage form not susceptible to abuse by mucosal membrane administration providing a plasma concentration that does not exceed the therapeutic concentration.

2. An oral pharmaceutical dosage form not susceptible to abuse by respiratory mucosal membrane administration providing a plasma concentration that does not exceed the therapeutic concentration.

3. An oral pharmaceutical dosage form not susceptible to abuse by mastication providing a plasma concentration that does not exceed the therapeutic concentration.

4. An oral pharmaceutical dosage form not susceptible to abuse by injection of an illicit extract of said oral dosage form providing a plasma concentration that does not exceed the therapeutic concentration.

5. An oral pharmaceutical dosage form not susceptible to abuse by mucosal membrane administration providing a plasma concentration that does not exceed the therapeutic concentration comprising a complex of a controlled substance and an ion exchange resin.

6. An oral pharmaceutical dosage form not susceptible to abuse by respiratory mucosal membrane administration providing a plasma concentration that does not exceed the therapeutic concentration comprising a complex of a controlled substance and an ion exchange resin.

7. An oral pharmaceutical dosage form not susceptible to abuse by mastication providing a plasma concentration that does not exceed the therapeutic concentration comprising a complex of a controlled substance and an ion exchange resin.

8. An oral pharmaceutical dosage form not susceptible to abuse by injection of an illicit extract of said oral dosage form providing a plasma concentration that does not exceed the therapeutic concentration comprising a complex of a controlled substance and an ion exchange resin.

9. An oral pharmaceutical dosage form according to Claim 5, wherein said controlled substance is selected from the group consisting of Schedule, II, III, IV or V controlled substances .

10. An oral pharmaceutical dosage form according to Claim 6, wherein said controlled substance is selected from the group consisting of Schedule, II, III, IV or V controlled substances .
